(19) Europäisches Patentamt
European Patent Office
Office européen des brevets

(11) Veröffentlichungsnummer: **0 004 608**
**A1**

(12) **EUROPÄISCHE PATENTANMELDUNG**

(21) Anmeldenummer: **79100876.6**

(22) Anmeldetag: **23.03.79**

(51) Int. Cl.²: **C 07 D 239/10**
C 07 F 9/65, C 07 D 403/04

(30) Priorität: **07.04.78 DE 2815061**

(43) Veröffentlichungstag der Anmeldung:
**17.10.79 Patentblatt 79/21**

(84) Benannte Vertragsstaaten:
**BE CH DE FR GB IT**

(71) Anmelder: **BASF Aktiengesellschaft**
**Carl-Bosch-Strasse 38**
**D-6700 Ludwigshafen(DE)**

(72) Erfinder: **Petersen, Harro, Dr.**
**Kalmitstrasse 23**
**D-6710 Frankenthal(DE)**

(54) **4-Acylamido-hexahydropyrimidine und Verfahren zu ihrer Herstellung.**

(57) Neue 4-Acylamido-hexahydropyrimidine und ein Verfahren zu ihrer Herstellung durch Umsetzung von Hexahydropyrimidinen mit Carbonsäureamiden bei Temperaturen über 100°C.

Die nach dem Verfahren der Erfindung herstellbaren 5,5-di-substituierten 4-Ureido-hexahydropyrimidine sind wertvolle Ausgangsstoffe für die Herstellung von Farbstoffen, Schädlingsbekämpfungsmitteln und Hilfsmitteln in der Textilchemie, Lackharzchemie, Holzwerkstoffindustrie.

EP 0 004 608 A1

BASF Aktiengesellschaft O.Z. 0050/033111

## 4-Acylamido-hexahydropyrimidine und Verfahren zu ihrer Herstellung

Die Erfindung betrifft neue 4-Acylamido-hexahydropyrimidine und ein Verfahren zu ihrer Herstellung durch Umsetzung von Hexahydropyrimidinen mit Carbonsäureamiden bei Temperaturen über 100°C.

Es ist aus den deutschen Offenlegungsschriften 16 70 261 und 16 70 262 bekannt, daß man 4-Hydroxy(4-Alkoxy)-2-oxo-hexahydropyrimidine mit Carbaminsäureestern in Gegenwart einer Säure bei Temperaturen zwischen 0 und 100°C zu 2-Oxo-hexahydropyrimidyl-4-N-carbamidsäureestern umsetzen kann.

Es ist bekannt, daß man durch Cyclokondensation von Harnstoffen mit einem Mol Formaldehyd und einem oder 2 Mol eines CH-aciden Aldehyds wie Isobutylraldehyd in Gegenwart von Säuren zu 4-Hydroxy-2-oxo-hexahydropyrimidinen gelangt, z.B.:

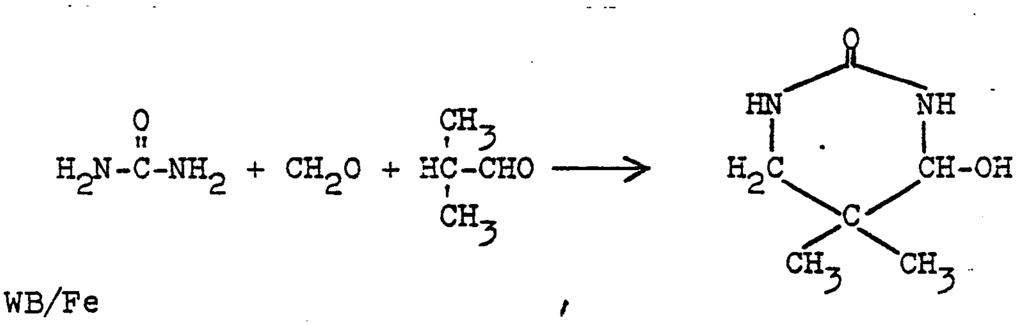

WB/Fe

Synthesis, Band 1973, Seiten 243 bis 292, insbesondere Seite 262 und 263). Diese Umsetzungen verlaufen mit befriedigenden Ausbeuten nur in den Fällen, in denen ein CH-acider Aldehyd eingesetzt wird, der in $\alpha$-Stellung zwei Substituenten aufweist. Setzt man CH-acide Aldehyde ein, die in $\alpha$-Stellung keinen (Acetaldehyd) oder nur einen Substituenten (n-Aldehyde) aufweisen, so erhält man bei der sauren Kondensation mit Harnstoffen 2-Oxo(thiono)-4-ureido-hexahydropyrimidine (IV) oder Dioxo(thiono)-decahydropyrimidopyrimidine (V):

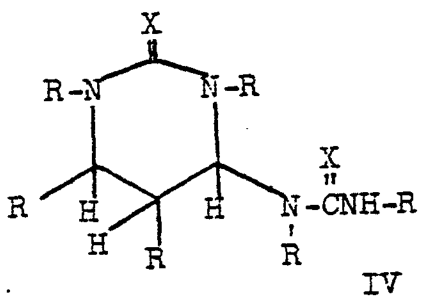
IV

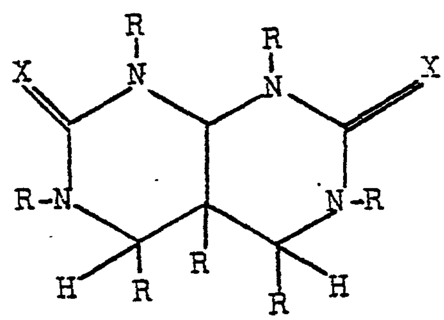
V

X = O, S
R = H, Alkyl

Bei Einsatz derartiger CH-acider Aldehyde bleibt die Reaktion nicht bei den 4-Hydroxy-Derivaten stehen (Synthesis Band 1973, Seiten 261 bis 280).

Es wurde nun gefunden, daß man 4-Acylamido-hexahydropyrimidine der Formel

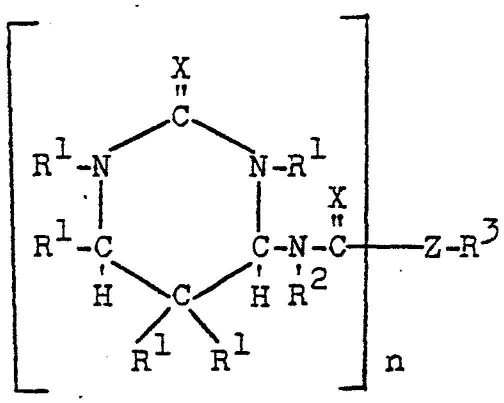 I,

worin die einzelnen Reste $R^1$, $R^2$ und $R^3$ gleich oder verschieden sein können und jeweils ein Wasserstoffatom, einen aliphatischen, cycloaliphatischen, araliphatischen oder aromatischen Rest bedeuten, $R^2$ und $R^3$ auch zusammen mit der benachbarten Carbonamidogruppe für Glieder eines heterocyclischen Ringes stehen können, die einzelnen Reste X gleich oder verschieden sein können und jeweils ein Sauerstoffatom oder ein Schwefelatom bedeuten, n 1 oder 2 bezeichnet, Z für eine Einfachbindung, wenn n 1 bedeutet, und für 2 Einfachbindungen, wenn n 2 bezeichnet, steht, vorteilhaft erhält, wenn man Hexahydropyrimidine der Formel

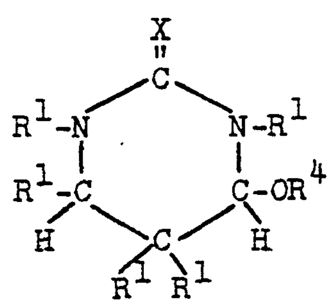 II,

worin $R^1$ und X die vorgenannte Bedeutung besitzen und $R^4$ ein Wasserstoffatom oder einen aliphatischen Rest bezeichnet, darüber hinaus $R^4$ und der in 3-Stellung sitzende Rest $R^1$ zusammen mit der diese beiden Reste verbindenden Aminalgruppe $-O-\underset{H}{\overset{|}{C}}-\overset{|}{N}-$ auch für Glieder eines heterocyclischen

Ringes stehen können, mit Carbonsäureamiden der Formel

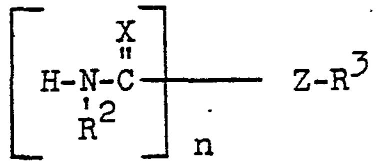 III,

worin $R^2$, $R^3$, n, Z und X die vorgenannte Bedeutung besitzen, bei einer Temperatur oberhalb von 100°C umsetzt.

Weiterhin wurden die neuen 4-Acylamido-hexahydropyrimidine der Formel

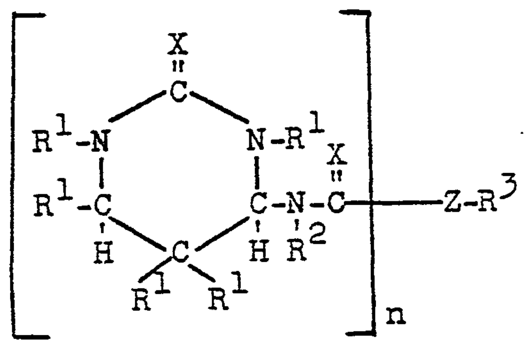 I,

worin die einzelnen Reste $R^1$, $R^2$ und $R^3$ gleich oder verschieden sein können und jeweils ein Wasserstoffatom, einen aliphatischen, cycloaliphatischen, araliphatischen oder aromatischen Rest bedeuten, $R^2$ und $R^3$ auch zusammen mit der benachbarten Carbonamidogruppe für Glieder eines heterocyclischen Ringes stehen können, die einzelnen Reste X gleich oder verschieden sein können und jeweils ein Sauerstoffatom oder ein Schwefelatom bedeuten, n 1 oder 2 bezeichnet, Z für eine Einfachbindung, wenn n 1 bedeutet, und für 2 Einfachbindungen, wenn n 2 bezeichnet, steht, gefunden.

Die Umsetzung kann für den Fall der Verwendung von Acetamid un von 2-Oxo-4-hydroxy-5,5-dimethyl-6-isopropyl-hexahydropyrimidin durch die folgenden Formeln wiedergegeben

werden.

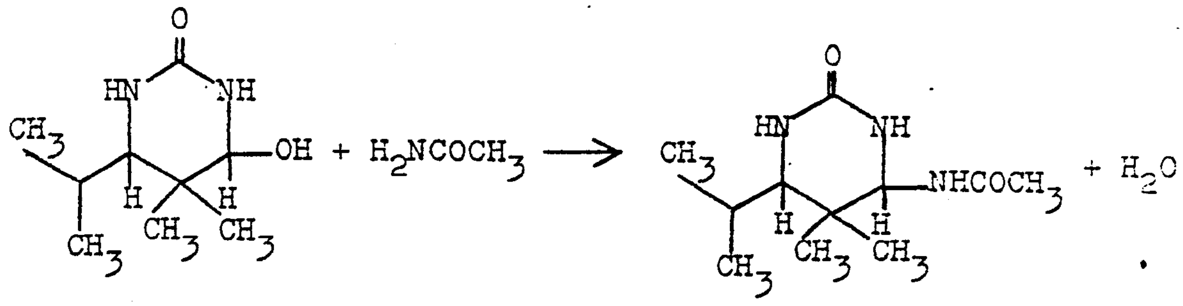

Im Hinblick auf den Stand der Technik liefert das Verfahren nach der Erfindung auf einfachem und wirtschaftlichem Wege eine große Zahl von bisher nicht beschriebenen 4-Acylamido-hexahydropyrimidinen in hoher Ausbeute und Reinheit. Man braucht nicht in Gegenwart von Säure umzusetzen. Da keine Säurekatalyse benötigt wird, entfällt die Neutralisation und die Abtrennung von Salzen. Zersetzungs- und Nebenreaktionen der Ausgangs- und Endstoffe werden vermieden. Die nach dem erfindungsgemäßen Verfahren herstellbaren Heterocyclen besitzen überraschend eine sehr hohe Temperaturstabilität. Während stickstoffhaltige Heterocyclen bei Temperatureinwirkung von über 100°C meist leicht bis stark verfärbt werden, bleiben die erfindungsgemäßen Endstoffe I praktisch farblos. Alle diese vorteilhaften Ergebnisse sind im Hinblick auf den Stand der Technik überraschend. Die Ausgangsstoffe II und III können in stöchiometrischer Menge oder im Überschuß jeder Komponente zur anderen, vorzugsweise in einem Verhältnis von 1 bis 1,5, insbesondere 1 bis 1,1 Mol Ausgangsstoff III je Ausgangsstoff II, umgesetzt werden. Bevorzugte Ausgangsstoffe II und III und dementsprechend bevorzugte Endstoffe I sind solche, in deren Formeln die einzelnen Reste $R^1$, $R^2$ und $R^3$ gleich oder verschieden sein können und jeweils ein Wasserstoffatom, einen Alkylrest oder Hydroxyalkylrest mit jeweils 1 bis 7 Kohlenstoffatomen, einen Alkenylrest mit 2 bis 7 Kohlen-

stoffatomen, einen Cyclohexanonylrest, einen Cyclohexylrest, einen Alkylarylrest oder Aralkylrest mit 7 bis 12 Kohlenstoffatomen oder einen Phenylrest bedeuten, $R^2$ und $R^3$ auch zusammen mit der benachbarten Carbonamidogruppe für Glieder eines 5-, 6- oder 7-gliedrigen, heterocyclischen Ringes stehen können, die einzelnen Reste X gleich oder verschieden sein können und jeweils ein Sauerstoffatom oder ein Schwefelatom bedeuten, $R^4$ ein Wasserstoffatom oder einen Alkylrest mit 1 bis 7 Kohlenstoffatomen bezeichnet, $R^4$ und der in 3-Stellung sizende Rest $R^1$ zusammen mit der diese beiden Reste verbindenden Aminalgruppe $-O-\underset{\underset{H}{|}}{\overset{|}{C}}-\overset{|}{N}-$ auch für Glieder eines 5- oder 6-gliedrigen, heterocyclischen Ringes stehen können. Es können Monocarbonsäureamide oder Dicarbonsäureamide als Ausgangsstoffe III verwendet und dementsprechend Mono- oder Di-[4-Acylamidohexahydropyrimidine] als Endstoffe I erhalten werden. Entsprechend ist Z eine Einfachbindung und n = 1 im Falle der Umsetzung von Monocarbonsäureamiden. Werden Dicarbonsäureamide III umgesetzt, so ist n = 2 und Z 2 Einfachbindungen und der die 2 Dicarbonamido-gruppen tragende Rest $R^3$ bevorzugt ein Alkylenrest mit 1 bis 7 Kohlenstoffatomen, ein Alkenylenrest mit 2 bis 7 Kohlenstoffatomen, ein Cyclohexylenrest, ein Aralkylenrest oder Alkylarylenrest mit 7 bis 12 Kohlenstoffatomen, ein Alkylarylalkylenrest mit 8 bis 12 Kohlenstoffatomen, ein Phenylenrest. Die vorgenannten Reste und Ringe können noch durch unter den Reaktionsbedingungen inerte Gruppen, z.B. Alkylgruppen mit 1 bis 4 Kohlenstoffatomen, substituiert sein.

So sind z.B. folgende Hexahydropyrimidine als Ausgangsstoffe II geeignet: 4-Hydroxy-2-oxo-hexahydropyrimidin; 5,5-Dimethyl-, 5,5-Diäthyl-, 5,5-Di-n-propyl-, 5,5-Diisopropyl-, 5,5-Di-n-butyl-, 5,5-Di-sek.-butyl-, 5,5-Diisobutyl-, 5,5-Di-tert.-butyl-, 5,5-Dihexyl-, 5,5-Diheptyl-,

5,5-Dipentyl-, 5-Phenyl-5-methyl-, 5-Benzyl-5-methyl-, 5-Cyclohexyl-5-methyl-, 5-Butyl-5-äthyl-, 5-Methyl-5-isopropyl-4-hydroxy-2-oxo-hexahydropyrimidin; entsprechende, in 1-, 3- und/oder 6-Stellung durch die Methyl-, Äthyl-, n-Propyl-, Isopropyl-, n-Butyl-, Isobutyl-, Hydroxyäthyl-, sek.-Butyl-, tert.-Butyl-, Heptyl-(1)-, Heptyl-(3)-, Hexyl-, Pentyl-, Phenyläthyl-(1)-, Phenyläthyl(2)-, Cyclohexyl-, Benzyl-, Phenyl-gruppe substituierte und in 5-Stellung nicht, einfach oder zweifach durch vorgenannten Gruppen substituierte 4-Hydroxy-2-oxo-hexyhydropyrimidine; homologe 4-Methoxy-, 4-Äthoxy-, 4-n-Propoxy-, 4-Isopropoxy-, 4-n-Butoxy-, 4-Isobutoxy-, 4-sek.-Butoxy-, 4-tert.-Butoxy-2-oxo-hexahydropyrimidine; entsprechende, in vorgenannten Stellungen unsubstituierte und/oder substituierte 2-Thiono-hexahydropyrimidine.

Besonders bevorzugte Ausgangsstoffe II sind:

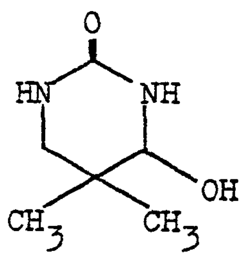 , 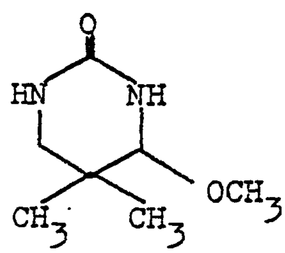 , 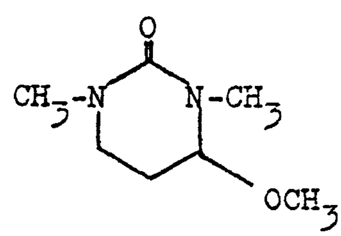 ,

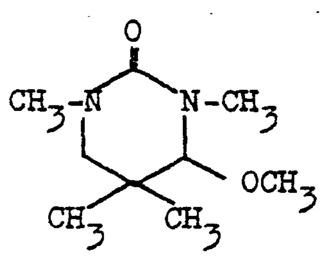 , 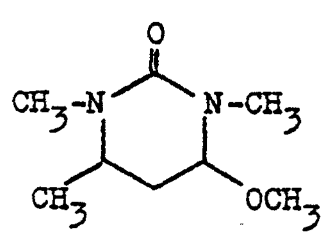 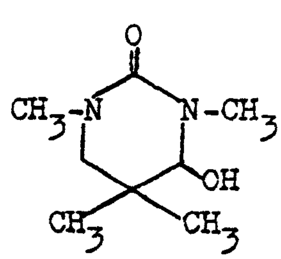 ,

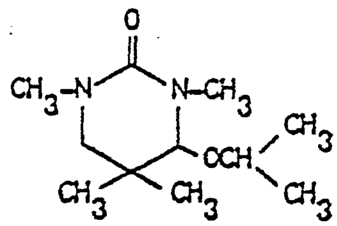 ,

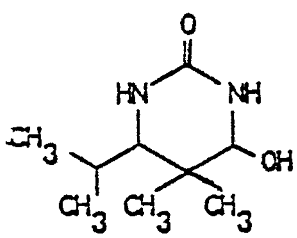 , 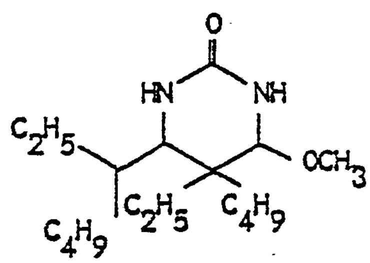 , 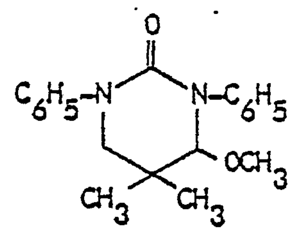 ,

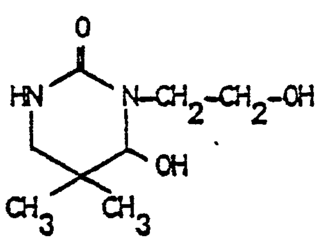 , 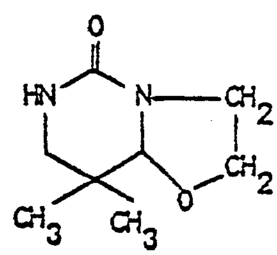 , 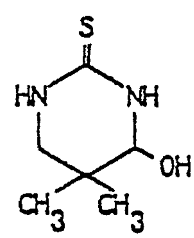 ,

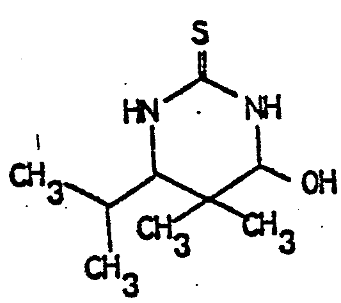 , 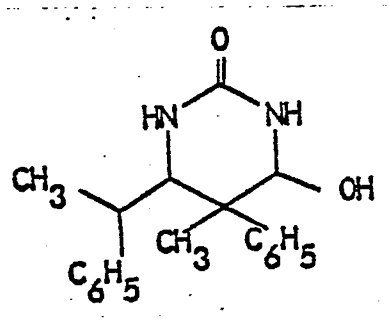

Geeignete Carbonsäureamide III sind beispielsweise: Formamid, Monomethyl-(N)-formamid, Monphenyl-(N)-formamid, Monostearyl-(N)-formamid, Monobenzyl-, Monocyclohexyl-, Monocyclopentyl-, Monoäthyl-, Monopropyl-, Monoisopropyl-, Monobutyl-, Mono-sek.-butyl-, Mono-tert.-butyl-, Mono-n-butyl-(N)-formamid; analoge, am Stickstoffatom unsubstituierte oder durch vorgenannte Reste monosubstituierte und in α-Stellung oder ω-Stellung zur Carbonylgruppe unsubstituierte oder durch die Hydroxy-, Methoxy-, Äthoxy-, Propoxy-, Isopropoxy-, Butoxy-, Isobutoxy-, sek.-Butoxy-, tert.-Butoxy-gruppe substituierte oder in ω-Stellung durch die Phosphonogruppe 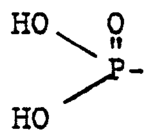 -, die Dimethoxy-, Diäthoxy-, Dipropoxy, Diisopropoxy-, Dibutoxy-, Di-sek.-butoxy-, Diisobutoxy-, Di-tert.-butoxy-phosphonogruppe substituierte Carbonsäureamide der Essigsäure, Propionsäure, Buttersäure, Isobuttersäure, Valeriansäure, Isovaleriansäure, Capronsäure, Caprylsäure, Önanthsäure, Caprinsäure, Acrylsäure, α-Methylacrylsäure, Cyclohexancarbonsäure, Benzoesäure, Phenylessigsäure, Cyclohexanon-2-carbonsäure; in vorgenannter Weise unsubstituierte, an einem oder beiden Stickstoffatomen monosubstituierte und/oder an einem oder beiden in α-Stellungen zu den Carbonamidgruppen substituierte Dicarbonsäureamide der Malonsäure, Bernsteinsäure, Glutarsäure, Adipinsäure, Pimelinsäure, Korksäure, Azelainsäure; das Lactam der ω-Aminobuttersäure, -valeriansäure, -capronsäure; entsprechend substituierte Thiocarbonsäureamide.

Bevorzugt sind folgende Ausgangsstoffe III:

$H-CONH_2$

$CH_3CONH_2$

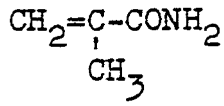

$CH_3OCH_2CH_2CONH_2$

$CH_2=CH-CONH_2$

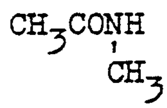

$CH_3CSNH_2$

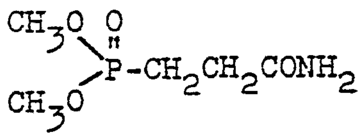

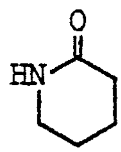

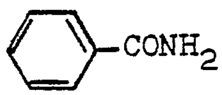

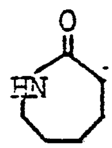

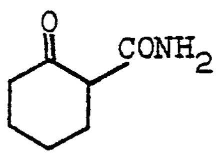

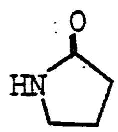

$H_2NCOCH_2CH_2CH_2CH_2CONH_2$

Die Umsetzung wird bei einer Temperatur oberhalb von 100°C, zweckmäßig von 100 bis 150°C, vorteilhaft zwischen 120 und 150°C, vorzugsweise von 121 bis 140°C, insbesondere von 125 bis 140°C, drucklos oder unter Druck, kontinuierlich oder diskontinuierlich durchgeführt. Man kann ohne Lösungsmittel umsetzen; zweckmäßig verwendet man aber unter den Reaktionsbedingungen inerte Lösungsmittel, insbesondere mit Siedepunkten über 100°C. Bei Flüssigkeiten mit Siedepunkten unter 100°C muß die Reaktion in geschlossenen Gefäßen unter Druck durchgeführt werden. Als Lösungsmittel kommen z.B. in Frage: aromatische Kohlenwasserstoffe, z.B.

Toluol, Äthylbenzol, o-, m-, p-Xylol, Isopropylbenzol, Methylnaphthalin; und entsprechende Gemische. Bevorzugte Lösungsmittel sind Toluol und Xylole. Es ist nicht erforderlich, daß die Ausgangsstoffe und Endstoffe in diesen Lösungsmitteln löslich sind. Die Unlöslichkeit der Endstoffe ermöglicht eine besonders leichte Aufarbeitung. Ferner muß das gebildete Reaktionswasser nicht unbedingt abgeführt werden. Es ist jedoch vorteilhaft, das Wasser durch azeotrope Destillation abzutrennen. Zweckmäßig verwendet man das Lösungsmittel in einer Menge von 400 bis 10 000 Gewichtsprozent, vorzugsweise von 400 bis 1 000 Gewichtsprozent, bezogen auf Ausgangsstoffe II. Die Umsetzung kann zweckmäßig in Abwesenheit von Lösungsmitteln durchgeführt werden, wenn eine oder beide Ausgangskomponenten oder der Endstoff flüssig oder bei der Reaktionstemperatur schmelzbar sind.

Die Reaktion wird schon aus Gründen des einfacheren bzw. wirtschaftlicheren Betriebs zweckmäßig ohne Zusatz von Säure durchgeführt. Gegebenenfalls kann man aber Säure, z.B. in Gestalt von Schwefelsäure, zweckmäßig in katalytischen Mengen, bevorzugt in Mengen von 0,005 bis 0,01 Äquivalenten Säure je Ausgangsstoff II, oder auch Basen, z.B. in Gestalt von Natronlauge, zweckmäßig in katalytischen Mengen, bevorzugt in Mengen von 0,005 bis 0,01 Äquivalenten Base je Ausgangsstoff II, zusetzen.

Die Reaktion kann wie folgt durchgeführt werden: Ein Gemisch der Ausgangsstoffe II und III und zweckmäßig Lösungsmittel wird während 0,25 bis 3 Stunden bei der Reaktionstemperatur gehalten. Dann wird der Endstoff aus dem Gemisch in üblicher Weise, z.B. durch Kristallisation und Filtration, abgetrennt.

Die nach dem Verfahren der Erfindung herstellbaren 5,5-disubstituierten 4-Ureido-hexahydropyrimidine sind wertvolle Ausgangsstoffe für die Herstellung von Farbstoffen, Schädlingsbekämpfungsmitteln und Hilfsmitteln in der Textilchemie, Lackharzchemie, Holzwerkstoffindustrie. Ihre N-Methylol- und N-Alkoxymethylverbindungen sind als reaktive Vernetzer in der Textilchemie, Lackharzchemie, Holzwerkstoffindustrie vorteilhaft verwendbar. So kann man beispielsweise die Trimethylolverbindung des 2-Oxo-5,5-dimethyl-hexahydropyrimidyl-(4)-N-carbamoyläthanphosphonsäuredimethylester zur waschpermanenten Flammschutzausrüstung und gleichzeitigen Knitterfreiausrüstung von Baumwollgeweben verwenden. Hierzu wird beispielsweise ein Baumwollköpergewebe in einer wäßrigen Flotte, die in 1 000 Volumenteilen Flüssigkeit 350 Teile der Verbindung

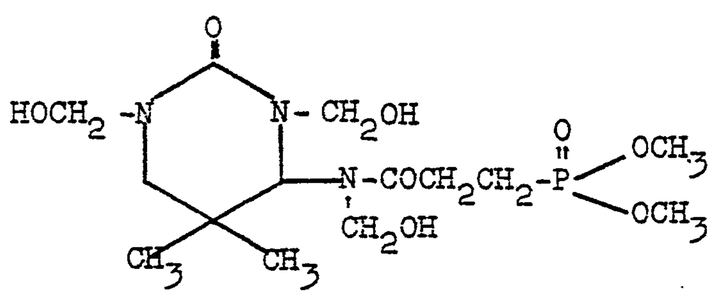

und 25 Teile einer 50-gewichtsprozentigen Phosphorsäure enthält, auf eine Flottenaufnahme von 100 Prozent foulardiert, bei 110°C getrocknet und 6 Minuten bei 170°C kondensiert. Das so ausgerüstete Gewebe zeigt einen hohen Flammschutz.

Die in den Beispielen genannten Teile bedeuten Gewichtsteile.

Beispiel 1

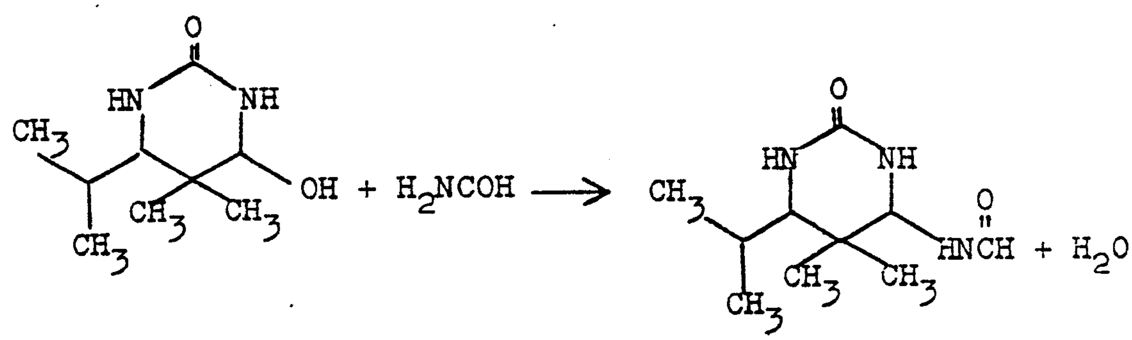

In einer Rührapparatur mit Rührer, Wasserabscheider, Rückflußkühler und Heizung werden 93 Teile 2-Oxo-4-hydroxy-5,5-dimethyl-6-isopropyl-hexahydropyrimidin zusammen mit 23 Teilen Formamid und 350 Teilen Xylol 40 Minuten auf 137°C erwärmt. Bei dieser Rückflußtemperatur werden in 40 Minuten 8,9 Teile Wasser aus dem Reaktionsgemisch durch azeotrope Destillation über den Wasserabscheider ausgekreist. Der Endstoff wird nach dem Abkühlen durch Filtration abgetrennt. Nach Trocknung werden 102 Teile 2-Oxo-4-N-formamido-5,5-dimethyl-6-isopropyl-hexahydropyrimidin in kristalliner Form erhalten. Das entspricht einer Ausbeute von 96 % der Theorie. Schmelzpunkt (aus Methanol) 203 bis 204°C.

Beispiel 2

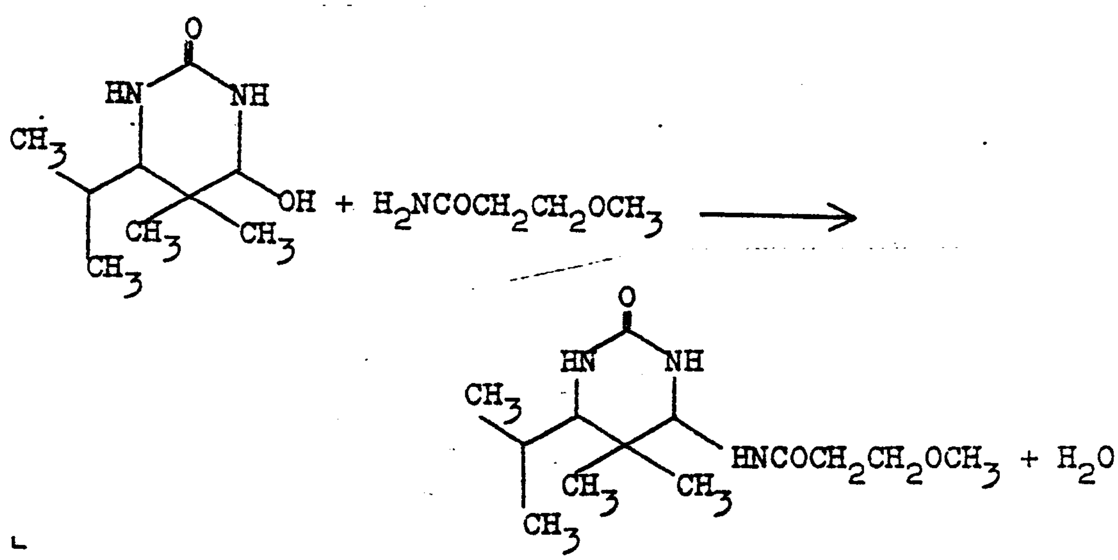

93 Teile 2-Oxo-4-hydroxy-5,5-dimethyl-6-isopropyl-hexahydropyrimidin, 51,5 Teile ß-Methoxypropionsäureamid und 300 Teile Xylol werden in der in Beispiel 1 beschriebenen Apparatur eine Stunde bei Rückflußtemperatur (138 bis 140°C) erwärmt. 9 Teile Wasser werden ausgekreist. Man kühlt und filtriert das Gemisch, engt das Filtrat ein und filtriert den ausgefallenen Endstoff. Es werden 134 Teile 2-Oxo-4-(ß-methoxypropionsäureamido)-5,5-dimethyl-6-isopropyl-hexahydropyrimidin erhalten. Das entspricht einer Ausbeute von 99 % der Theorie. Fp (aus Aceton) 171°C.

Beispiel 3

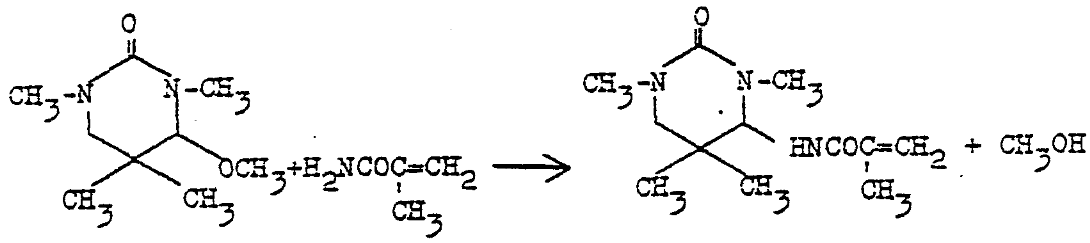

Analog Beispiel 1 werden 372 Teile 2-Oxo-4-methoxy-1,3,5,5-tetramethyl-hexahydropyrimidin, 170 Teile Methacrylamid und 800 Teile Xylol unter Rühren 10 Minuten auf 110 bis 120°C am Rückfluß erhitzt. Nach einer Gesamtreaktionsdauer von 70 Minuten werden bei 100 bis 120°C 61 Teile Methanol in der Vorlage aufgefangen. Nach Destillation des Xylols und Abkühlen auf Raumtemperatur wird der Endstoff abfiltriert und getrocknet. Es werden 462 Teile 2-Oxo-4-(N-methacrylamido)-1,3,5,5-tetramethylhexahydropyrimidin erhalten. Das entspricht einer Ausbeute von 97 % der Theorie. Schmelzpunkt (Essigsäureäthylester) 148 bis 149°C.

Beispiel 4

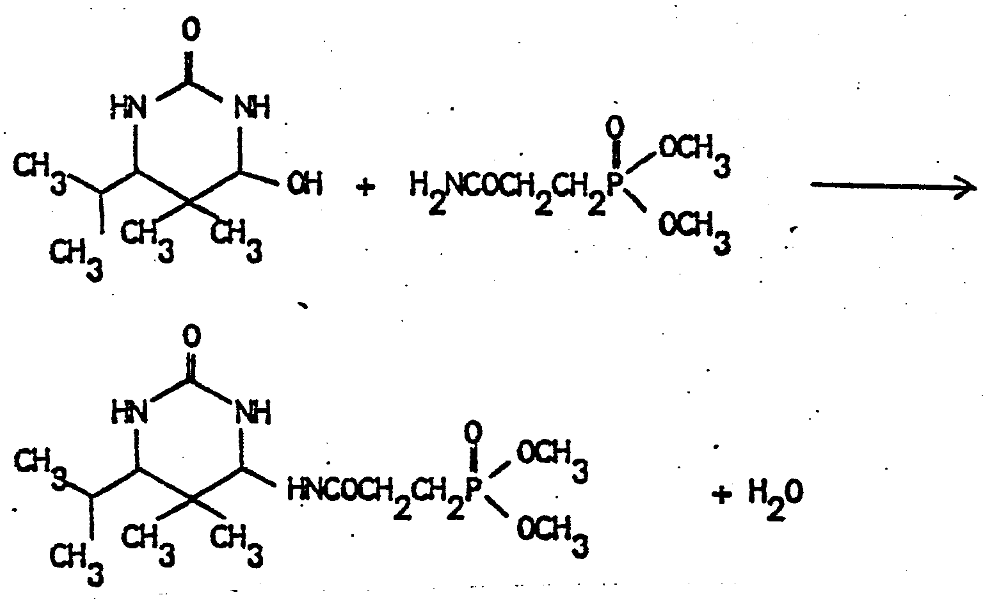

Analog Beispiel 1 wird das Gemisch aus 93 Teilen 2-Oxo-4-hydroxy-5,5-dimethyl-6-isopropyl-hexahydropyrimidin und 90,5 Teilen Carbamoyläthanphosphorsäuredimethylester in 500 Teilen Xylol eine Stunde bei Rückflußtemperatur (136 bis 138°C) erwärmt, wobei 9 Teile Reaktionswasser über den Wasserabscheider abgetrennt werden. Nach Abkühlen des Gemisches wird der kristallin ausfallende Endstoff abfiltriert und getrocknet. Es werden 171 Teile (2-Oxo-5,5-dimethyl-6-isopropyl-hexahydropyrimidyl)-(4)-N-carbamoyläthanphosphonsäuredimethylester erhalten. Das entspricht einer Ausbeute von 98 % der Theorie. Schmelzpunkt (Äthanol) 120°C.

Beispiel 5

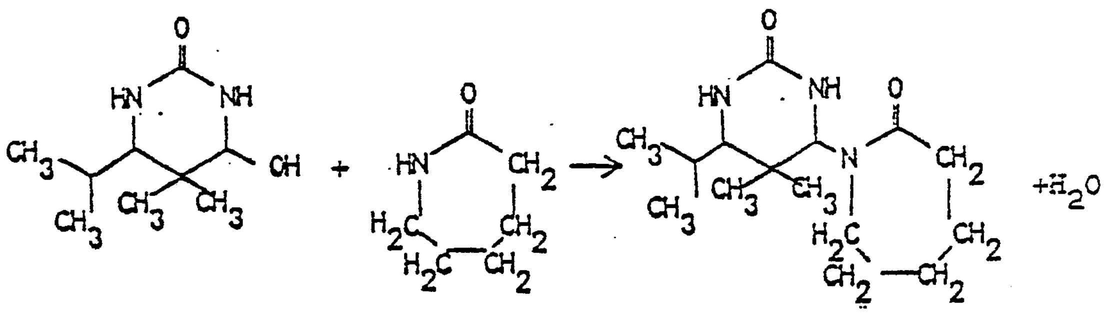

93 Teile 2-Oxo-4-hydroxy-5,5-dimethyl-6-isopropyl-hexahydropyrimidin werden zusammen mit 56,5 Teilen Caprolactam in 350 Teilen Xylol analog Beispiel 1 zwei Stunden bei Rückflußtemperatur (135 bis 140°C) erhitzt, wobei 9 Teile Reaktionswasser über dem Wasserabscheider abgetrennt werden. Anschließend wird das Lösungsmittel unter vermindertem Druck abdestilliert. Es werden 137 Teile (2-Oxo-5,5-dimethyl-6-isopropyl-hexahydropyrimidyl-(4))-N-caprolactam vom Fp 146 bis 148°C (unter Zersetzung) erhalten. Das entspricht einer Ausbeute von 97,5 % der Theorie.

Beispiel 6

Führt man die Umsetzung analog Beispiel 4 mit 72 Teilen 2-Oxo-4-hydroxy-5,5-dimethyl-hexahydropyrimidin durch, so erhält man 143 Teile (2-Oxo-5,5-dimethyl-hexahydropyrimidyl)-(4)-N-carbamoyläthanphosphonsäuredimethylester vom Fp 103 bis 106°C.

Patentansprüche

1. Verfahren zur Herstellung von 4-Acylamido-hexahydropyrimidinen der Formel

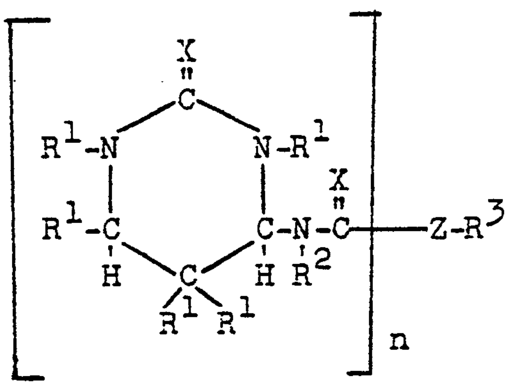

I,

worin die einzelnen Reste $R^1$, $R^2$ und $R^3$ gleich oder verschieden sein können und jeweils ein Wasserstoffatom, einen aliphatischen, cycloaliphatischen, araliphatischen oder aromatischen Rest bedeuten, $R^2$ und $R^3$ auch zusammen mit der benachbarten Carbonamidogruppe für Glieder eines heterocyclischen Ringes stehen können, die einzelnen Reste X gleich oder verschieden sein können und jweils ein Sauerstoffatom oder ein Schwefelatom bedeuten, n 1 oder 2 bezeichnet, Z für einen Einfachbindung, wenn n 1 bedeutet, und für 2 Einfachbindungen, wenn n 2 bezeichnet, steht, dadurch gekennzeichnet, daß man Hexahydropyrimidine der Formel

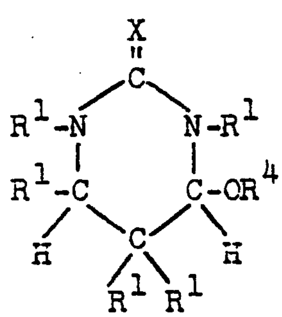

II,

worin $R^1$ und X die vorgenannte Bedeutung besitzen und $R^4$ ein Wasserstoffatom oder einen aliphatischen Rest bezeichnet, darüber hinaus $R^4$ und der in 3-Stellung sitzende Rest $R^1$ zusammen mit der diese beiden Reste verbindenden Aminalgruppe 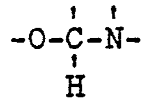 auch für Glieder eines heterocyclischen Ringes stehen können, mit Carbonsäureamiden der Formel

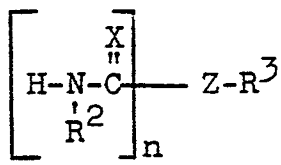 III,

worin $R^2$, $R^3$, n, Z und X die vorgenannte Bedeutung besitzen, bei einer Temperatur oberhalb von 100°C umsetzt.

2. 4-Acylamido-hexahydropyrimidine der Formel

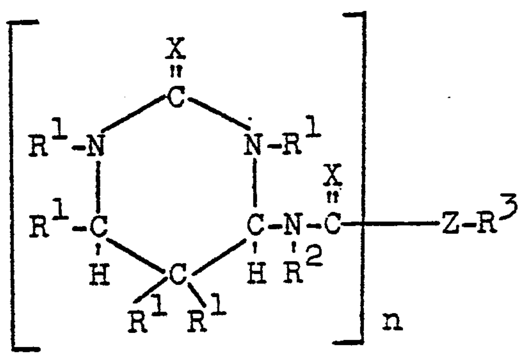 I,

worin die einzelnen Reste $R^1$, $R^2$ und $R^3$ gleich oder verschieden sein können und jeweils ein Wasserstoffatom, einen aliphatischen, cycloaliphatischen, araliphatischen oder aromatischen Rest bedeuten, $R^2$ und $R^3$ auch zusammen mit der benachbarten Carbonamidogruppe für Glieder eines heterocyclischen Ringes stehen können, die einzelnen Reste X gleich oder verschieden sein können und jeweils ein Sauerstoffatom oder ein Schwefelatom bedeuten,

n 1 oder 2 bezeichnet, Z für eine Einfachbindung, wenn n 1 bedeutet, und für 2 Einfachbindungen, wenn n 2 bezeichnet, steht.

Europäisches Patentamt

# EUROPÄISCHER RECHERCHENBERICHT

Nummer der Anmeldung
EP 79 10 0876

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| | Keine Entgegenhaltungen.<br>---- | | C 07 D 239/10<br>C 07 F 9/65<br>C 07 D 403/04 |
| | | | RECHERCHIERTE SACHGEBIETE (Int. Cl.²) |
| | | | C 07 D 239/10<br>C 07 F 9/65<br>C 07 D 403/04 |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Den Haag | 12-07-1979 | FRANCOIS |